(19) Europäisches Patentamt
European Patent Office
Office européen des brevets

(11) Veröffentlichungsnummer: **0 071 787**
**B1**

(12) **EUROPÄISCHE PATENTSCHRIFT**

(45) Veröffentlichungstag der Patentschrift: 21.11.85

(51) Int. Cl.[4]: B 01 J 23/89, C 07 C 29/136, C 07 C 29/14, C 07 C 33/03

(21) Anmeldenummer: 82106324.5

(22) Anmeldetag: 15.07.82

(54) **Neue Ruthenium/Kohle-Hydrierkatalysatoren, deren Herstellung und Verwendung zur selektiven Hydrierung von ungesättigten Carbonylverbindungen.**

(30) Priorität: 04.08.81 DE 3130805

(43) Veröffentlichungstag der Anmeldung: 16.02.83 Patentblatt 83/07

(45) Bekanntmachung des Hinweises auf die Patenterteilung: 21.11.85 Patentblatt 85/47

(84) Benannte Vertragsstaaten: CH DE FR GB IT LI NL

(56) Entgegenhaltungen:
EP-A- 0 007 101
EP-A- 0 024 648
DE-A- 2 650 046
DE-A- 3 019 582
GB-A- 2 024 643
US-A- 3 284 517

(73) Patentinhaber: BASF Aktiengesellschaft
Carl-Bosch-Strasse 38
D-6700 Ludwigshafen (DE)

(72) Erfinder: Horner, Michael, Dr.
Im Ritterbueschel 9
D-6730 Neustadt (DE)
Erfinder: Irgang, Matthias, Dr.
Andreas-Hofer-Weg 41
D-6900 Heidelberg (DE)

EP 0 071 787 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft mit Eisen dotierte Ruthenium/Kohlenstoffträger-Katalysatoren, deren Herstellung und deren Verwendung zur Herstellung von olefinisch ungesättigten Alkoholen durch selektive Hydrierung der entsprechenden Carbonylverbindungen in der Flüssigphase in Gegenwart von Edelmetallkatalysatoren sowie in Gegenwart eines tertiären Amins.

Aus der DE-A-29 34 251 ist ein Verfahren zur Herstellung von olefinisch ungesättigten Alkoholen, insbesondere von Geraniol und Nerol (E- und Z-3,7-Dimethyl-octa-2,6-dien-1-ol) durch selektive Hydrierung der entsprechenden α,β-ungesättigten Carbonylverbindungen in der Flüssigphase in Gegenwart von Edelmetallkatalysatoren, wie Ruthenium-, Rhodium-, Osmium-, Iridium- oder Platin-Katalysatoren sowie in Gegenwart eines tertiären Amins bekannt. Wie aus der Beschreibungseinleitung zu der genannten DE-A hervorgeht, ist die technische Herstellung der genannten ungesättigten Alkohole ziemlich problematisch, da entweder große Mengen an den sehr teuren Edelmetallkatalysatoren notwendig sind, diese auf aufwendige Weise vorbehandelt werden müssen, die Aktivität der Katalysatoren in vielen Fällen relativ schnell nachläßt und/oder die Selektivität der Katalysatoren zu wünschen übrig läßt. Die gemäß dieser DE-A vorgeschlagene Anwesenheit eines tertiären Amins im Reaktionsgemisch bringt mit relativ geringem technisch-wirtschaftlichen Aufwand eine wesentliche Erhöhung der Selektivität der Umsetzung sowie eine Verbesserung der Aktivität des Katalysators über lange Betriebszeiten mit sich. Jedoch weist auch dieses Verfahren, insbesondere bei Verwendung der kostengünstigen handelsüblichen Rutheniumkatalysatoren noch folgende Nachteile auf:

1. Die Selektivität der Rutheniumkatalysatoren ist noch nicht ganz befriedigend, insbesondere bei der Synthese von Geraniol.

2. Die Hydrierung stoppt nicht selektiv nach Aufnahme von 1 Mol Wasserstoff pro Mol Aldehyd auf der Stufe des Alkohols, was zu einem Anstieg der destillativ schwer entfernbaren Überhydrierungsprodukte Citronellol bzw. Tetrahydrogeraniol und somit zu einer Erschwerung der Reindarstellung der als Riechstoffe begehrten ungesättigten Alkohole führt.

3. Die Raum-Zeit-Ausbeuten im Hochdruckbereich sind noch unbefriedigend.

4. Die Hydrierzeiten im Druckbereich $< 50$ bar sind bei niedrigem Katalysatoreinsatz zu lang.

Es war daher die Aufgabe der Erfindung, für das an sich sehr vorteilhafte Verfahren der DE-A 29 34 251 einen Katalysator auf Basis von Ruthenium, dem mit Abstand billigsten Platinmetall, zu entwickeln, der die beschriebenen Nachteile bezüglich Hydrieraktivität und selektivem Hydrierstopp bei der Hydrierung der ungesättigten Aldehyde weitgehend vermeidet.

Ruthenium/Aktivkohle-Katalysatoren werden in technischem Maßstab hergestellt und kommerziell angeboten. Der Stand der Technik über Ru/C-Katalysatoren wird in der Monographie von J. D. Anderson « Structure of Metallic Catalysts » (London, N. York, San Francisco 1975) dargestellt. Neuere Arbeiten mit Angaben über die Herstellung von Ru/C-Katalysatoren fehlen.

Als Ausgangssubstanzen für Ru-Katalysatoren werden u. a.

$RuCl_3$ bzw. $RuCl_3 \cdot aq.$

$(NH_4)_2\ [RuCl_6]$

$(NH_4)_2\ [RuCl_5 \cdot H_2O]$

verwendet.

Für die Katalysatorherstellung sind verschiedene Methoden bekannt. Die wichtigsten sind:

1. Tränkung des Trägers mit einer löslichen Ru-Verbindung.

2. Kationenaustausch an geeigneten Trägern, ausgehend von $[Ru(NH_3)_4(NO)(OH)]^{2+}$ oder $[Ru(NH_3)_6]^{3+}$.

3. Ausfällung schwerlöslicher Ru-Verbindungen auf der Oberfläche des Trägers.

Zusätzlich erfolgt gleichzeitig oder anschließend die Reduktion des Katalysators. Für die Reduktion mit Wasserstoff werden in der Literatur (Orito, Y. und Sabi, O. Yoki Gosei Kagaku Kyokaishi *30* (1972) 6, 563-565) Temperaturen zwischen 250 und 500 °C angegeben.

Gegenstand der Erfindung ist ein Ruthenium/Kohle-Katalysator für die Herstellung von olefinisch ungesättigten Alkoholen durch selektive Hydrierung der entsprechenden ungesättigten Carbonylverbindungen in der Flüssigphase, enthaltend neben 0,1 bis 10 Gew.%, vorzugsweise 1 bis 6 Gew.% Ruthenium auf einen Kohlenstoffträger, vorzugsweise auf Aktivkohle, 0,1 bis 5 Gew.%, vorzugsweise 0,5 bis 1,5 Gew.% Eisen und hergestellt unter Beachtung der folgenden Maßnahmen:

a) Dotierung des Katalysators mit Eisen erst nach erfolgter Imprägnierung mit der Rutheniumverbindung und

b) Reduktion des Katalysators mit Wasserstoff unter intensiver Durchmischung bei 400 bis 600 °C insbesondere ein solcher Ruthenium/Kohle-Katalysator, bei dessen Herstellung die Reduktion des Katalysators mit Wasserstoff bei Temperaturen zwischen 500 und 600 °C vorgenommen wurde.

Gegenstand der Erfindung ist weiterhin ein Verfahren zur Herstellung dieser Ruthenium/Kohle-Katalysatoren für die Herstellung von olefinisch ungesättigten Alkoholen durch Imprägnieren des Kohlenstoffträgers mit einer Rutheniumverbindung und anschließende Reduktion des Katalysators mit Wasserstoff, das dadurch gekennzeichnet ist, daß man

a) den Katalysator nach Imprägnierung mit der Rutheniumverbindung mit 0,1 bis 5, vorzugsweise 0,5 bis 1,5 Gew.% an Eisen dotiert und

b) die Reduktion des Katalysators mit Wasserstoff unter intensiver Durchmischung bei 400 bis 600 °C durchführt,
insbesondere ein solches Verfahren, das dadurch gekennzeichnet ist, daß man die Reduktion des Katalysators mit Wasserstoff bei Temperaturen zwischen 500 und 600 °C durchführt.

Gegenstand der Erfindung ist weiterhin die Verwendung der oben beschriebenen Ruthenium/Kohle-Katalysatoren für die Herstellung von ungesättigten Alkoholen der allgemeinen Formel Ia

$$R^1-\overset{R^2}{\overset{|}{C}}=\overset{R^3}{\overset{|}{C}}-\overset{R^4}{\overset{|}{C}}H-OH \qquad (Ia)$$

in der $R^1$ Wasserstoff oder einen organischen Rest und $R^2$, $R^3$ und $R^4$ Wasserstoff oder eine $C_1$-$C_4$-Alkylgruppe bedeuten, durch selektive Hydrierung der entsprechenden α,β-ungesättigten Carbonylverbindungen IIa

$$R^1-\overset{R^2}{\overset{|}{C}}=\overset{R^3}{\overset{|}{C}}-\overset{R^4}{\overset{|}{C}}=O \qquad (IIa)$$

in der Flüssigphase in Gegenwart eines tertiären Amins.

Der erfindungsgemäße Hydrierkatalysator enthält neben Ruthenium und einem Kohlenstoffträger, wie Aktivkohle oder Ruß, vorzugsweise Aktivkohle, 0,1 bis 5 Gew.%, vorzugsweise 0,5 bis 1,5 Gew.% Eisen. Eine derartige Dotierung mit Eisen ist für Ru/C-Katalysatoren noch nicht bekannt.

Bekannt ist es, die Selektivität für die Hydrierung der Formylgruppe des Zimtaldehyds an Platinkatalysatoren dadurch zu verbessern, daß man noch andere Katalysatorkomponenten wie Eisen und Zink (BE-A 837 057), Eisen und Silber (US-A 3 284 517) oder Cobalt (DE-A 24 12 517) mitverwendet. Jedoch ist die Verwendung von den sehr teuren Edelmetallen wie Platin aus wirtschaftlichen Gründen nur dann vorteilhaft, wenn man den Katalysator in die Reaktionszone zurückführen kann, ohne daß wesentliche Änderungen der katalytischen Aktivität auftreten. Tatsächlich machte sich bei den genannten Katalysatorsystemen durch die Mitverwendung anderer Katalysatorkomponenten eine Verminderung der Katalysatorwirksamkeit bemerkbar, wodurch sich auch der industrielle Nutzen dieser Hydrierverfahren vermindert (vgl. DE-A 26 50 046, Seite 3). Es war daher sehr überraschend, daß man bei Verwendung eines Rutheniumkatalysators, dem vorteilhaftesten, da mit Abstand dem billigsten Platinmetall, eine selektivitätserhöhende Dotierung mit Eisen erzielen konnte, die nicht mit einer Aktivitätsverminderung verbunden war, sondern die sogar zu einer Erhöhung der Katalysatoraktivität führte, was sich beispielsweise dadurch zeigt, daß man bei Einsatz der gleichen Katalysatormenge die Hydrierzeiten drastisch vermindern kann (s. Beispiele 3, 7, 10 und 11 im Vergleich mit den Vergleichsbeispielen 3V, 7V, 10V und 11V), ja sogar kürzere Hydrierzeiten als mit der 2,5 fachen Menge eines undotierten Katalysators benötigt (siehe 4 im Vergleich mit 4V). Gleichzeitig mit der Erhöhung der Katalysatoraktivität wird eine verbesserte Selektivität und ein selektiver Hydrierstopp erzielt. Die Beispiele 5 und 9 zeigen ferner, daß die erfindungsgemäßen Katalysatoren auch im Druckbereich von 20 bar noch Hydrierungen in wirtschaftlichen Zeiten ermöglichen, während gleiche Mengen handelsübliche Katalysatoren bei diesen Drucken nur eine unvollständige Hydrierung ermöglichen.

Es wurde weiterhin überraschenderweise festgestellt, daß durch die erfindungsgemäße Eisendotierung auch eine hohe Lebensdauer der Ruthenium/C-Katalysatoren erzielt wurde, was sich durch eine hohe Zahl von Produktionschargen ausdrückt, die mit der gleichen Katalysatorfüllung auf gleich vorteilhafte Weise hydriert werden kann (siehe Beispiel 6 im Vergleich mit Beispiel 4).

Die genannten Katalysatorverbesserungen durch Eisendotierung lassen sich jedoch nur dann erhalten, wenn bei der Herstellung bestimmte Maßnahmen beachtet werden. So erwiesen sich eine gemeinsame Imprägnierung des Kohlenstoffträgers mit Rutheniumchlorid- und Eisenchlorid- bzw. Eisennitrat-Lösung als unwirksam, die so erhaltenen Katalysatoren zeigten geringere Aktivität als undotierte Muster. Überraschenderweise wurden verbesserte Katalysatoren nur dann erhalten, wenn die Eisendotierung erst nach der Imprägnierung mit der Rutheniumverbindung erfolgte.

Mit Vorteil arbeitet man so, daß man zunächst das Ruthenium in üblicher Weise auf den Kohlenstoffträger aufbringt und dann das so erhaltene und getrocknete Katalysatorvorprodukt mit Eisenoxid oder Eisen in Form von Eisenoxidpulver oder Eisenpulver vermischt. Während des Mischens oder nach dem Mischen wird das Katalysatorvorprodukt mit Wasserstoff reduziert.

Auch die Reduktionsbedingungen haben starken Einfluß auf die Katalysatoreigenschaften. So zeigte sich, daß Ru/C-Katalysatoren, die bei Temperaturen unterhalb von 400 °C — wie sie für die Reduktion von Edelmetallkatalysatoren allgemein üblich sind — reduziert wurden, eine wesentlich geringere Aktivität aufweisen. Die höchsten Hydrieraktivitäten an Ru/C-Katalysatoren wurden erzielt bei Katalysatoren, die bei Temperaturen zwischen 500 und 600 °C mit Wasserstoff reduziert wurden. Diese Temperatur liegt deutlich oberhalb der bisher üblichen Reduktionstemperatur für Edelmetall/C-Katalysatoren.

Weiterhin ist eine gute Durchmischung des Katalysators während der Reduktion mit Wasserstoff erforderlich. Alle Versuche zur Erzeugung eines hochaktiven Ru/C-Katalysators durch Reduktion in ruhender Schicht oder im Festbett verliefen negativ. Gute Erfolge traten dagegen bei Reduktion im Drehrohrofen auf.

Offenbar sind die oben angeführten Maßnahmen wesentlich, um eine geeignete Struktur der Ru/Fe-Kristallite auf bzw. in den Aktivkohlepartikeln zu erzeugen.

Zur weiteren Charakterisierung der erfindungsgemäßen Katalysatoren sei noch folgendes ausgeführt :

Die erfindungsgemäßen Katalysatoren enthalten im allgemeinen 0,1 bis 10 Gew.% Ruthenium auf einem Kohlenstoffträger wie Aktivkohle oder Ruß, vorzugsweise auf Aktivkohle. Recht gute Erfolge wurden mit Katalysatoren erzielt, die — wie die meisten handelsüblichen Edelmetall/-Kohle-Katalysatoren — 5 % Ruthenium auf Aktivkohle enthalten.

Bezogen auf den Metallgehalt und auf die Menge der Ausgangsverbindungen II setzt man das Ruthenium vorzugsweise in Mengen von 0,000 1 bis 0,1, besonders 0,001 bis 0,1 Gew.% ein.

Die BET-Oberfläche der Katalysatoren beträgt, entsprechend der zur Herstellung verwendeten Kohlenstoffträger für Aktivkohle etwa 600 bis 900, vorzugsweise etwa 700 bis 800 $m^2/g$ und für Ruß etwa 300 bis 700 $m^2/g$. Die Teilchengröße der Ru-Kristallite liegt zwischen 2 und 5 nm und entspricht damit den aus der Literatur für Ru/C-Katalysatoren bekannten Werten. Das Ru liegt in elementarer Form vor, wie sich aus ESCA-Aufnahmen ergab. Da der Katalysator in Gegenwart von Luft pyrophor sein kann, wird er vorteilhaft in Pulver, das 50 % $H_2O$ enthält, gehandhabt. Dieses Pulver ist noch gut rieselfähig, jedoch nicht staubend und nicht pyrophor. Die Angaben über die im Katalysator enthaltenen Gew.% an Ruthenium und Eisen beziehen sich in der vorliegenden Anmeldung immer auf die Trockenmasse des Katalysators.

Das spezielle Anwendungsgebiet der erfindungsgemäßen Katalysatoren liegt bei der Herstellung von ungesättigten Alkoholen der allgemeinen Formel Ia

$$R^1-\overset{R^2}{\overset{|}{C}}=\overset{R^3}{\overset{|}{C}}-\overset{R^4}{\overset{|}{C}}H-OH \qquad \text{(Ia)}$$

in der $R^1$ Wasserstoff oder einen organischen Rest und $R^2$, $R^3$ und $R^4$ Wasserstoff oder eine $C_1$-$C_4$-Alkylgruppe bedeuten, durch selektive Hydrierung von α,β-ungesättigten Carbonylverbindungen IIa

$$R^1-\overset{R^2}{\overset{|}{C}}=\overset{R^3}{\overset{|}{C}}-\overset{R^4}{\overset{|}{C}}=O \qquad \text{(IIa)}$$

unter den in der DE-A 29 34 251 beschriebenen Reaktionsbedingungen.

Besondere Bedeutung hat das erfindungsgemäße Verfahren auch hier für die bekanntermaßen technisch problematische Hydrierung von Citral IIb zu Geraniol bzw. Nerol (E-Ib bzw. Z-Ib), da hier Mehrfachhydrierung und Isomerisierung als Konkurrenzreaktionen auftreten.

Ebensogut ist das Verfahren aber auch auf die sonstigen Verbindungen IIa anwendbar, wobei es meistens auch die genannten Vorteile über die bisher bekannten Verfahren bietet, wie es fast durchweg für die definitionsgemäßen Verbindungen IIa zutrifft, deren olefinische Doppelbindung wegen ihrer Konjugation zur Carbonylgruppe besonders leicht hydriert wird.

Die Reste $R^1$ in den Verbindungen IIa können prinzipiell beliebig sein. Enthalten diese Reste weitere olefinische Doppelbindungen, so werden diese nicht angegriffen. Beispiele für $R^1$ sind Alkyl- und Alkenylreste mit 1 bis 20 C-Atomen und aromatische Reste wie die Phenylgruppe. Diese Reste können ihrerseits beispielsweise Alkylgruppen, Alkoxygruppen, Carbalkoxygruppen, Acylgruppen, Hydroxylgruppen, Carboxylgruppen, Nitrilgruppen, Aminogruppen und Halogen als Substituenten tragen. Da das Verfahrensprinzip von der Art der Substituenten $R^1$ bis $R^3$ nicht berührt wird, erübrigt sich eine gesonderte Aufzählung der hier möglichen Ausgangsverbindungen IIa. Dies gilt auch für die Ausgangsverbindungen II allgemein, deren wesentliches Kriterium die Anwesenheit einer oder mehrerer olefinischer Doppelbindungen ist, welche bei der Hydrierung der Carbonylgruppe nicht oder nicht nennenswert angegriffen werden sollen. Die sonstige chemische Natur spielt hier keine Rolle, jedoch handelt es sich in der Praxis meist um unverzweigte oder verzweigte ein- oder mehrfach ungesättigte Alkenale und Alkenole mit 4 bis 40 C-Atomen.

Die Hydrierung der Carbonylverbindungen IIa an den erfindungsgemäßen Katalysatoren wird in Gegenwart eines tertiären Amins durchgeführt.

Prinzipiell sind nach den bisherigen Beobachtungen jegliche tertiäre Amine geeignet, so daß es auf deren chemische Natur, sofern sie aufgrund funktioneller Gruppen nicht anderweitig mit den Reaktionspartnern reagieren können, nicht ankommt. Genannt seien beispielsweise

— aliphatische tertiäre Amine mit insgesamt 3 bis 30 C-Atomen, wie vor allem Trimethylamin sowie Triethylamin, Triethanolamin und Trihexylamin,
— cyclische tertiäre Amine wie N-Methylpiperidin, N-Methylmorpholin und N,N'-Dimethylpiperazin
— aliphatisch-cycloaliphatische tertiäre Amine wie N,N-Dimethylcyclohexylamin,
— aliphatisch-araliphatische tertiäre Amine wie N,N-Dimethylbenzylamin,
— aliphatisch-aromatische tertiäre Amine wie N,N-Dimethylanilin sowie
— heterocyclisch-aromatische tertiäre Amine wie Pyridin und Chinolin.

Aus wirtschaftlichen Gründen wird man im übrigen möglichst billige Amine verwenden, deren Siedepunkt entweder deutlich tiefer oder deutlich höher ist, als der des Verfahrensproduktes, da sich in diesen Fällen entweder die Amine oder die Verfahrensprodukte leicht aus dem Reaktionsgemisch abdestillieren lassen.

Die Menge der Amine beträgt vorzugsweise 25 bis 40 Gew.% des Einsatzstoffes II.

Es empfiehlt sich, die Reaktion in Gegenwart eines Lösungsmittels vorzunehmen. Die Mengen an Lösungsmittel liegen im allgemeinen zwischen 10 und 300, vorzugsweise 25 und 150 Gew.% von II. Als Lösungsmittel eignen sich alle inerten Flüssigkeiten, in denen sowohl I und II als auch das mitverwendete tertiäre Amin löslich sind. In Betracht kommen beispielsweise die tertiären Amine selber sowie zusätzlich Alkohole wie Methanol und Ethanol, Ether, Aceton und unter den Reaktionsbedingungen flüssige Kohlenwasserstoffe wie Hexan und Cyclohexan. Bevorzugt wird Methanol, und zwar vor allem, wenn man Trimethylamin als Base verwendet, da sich in diesem Falle die Aufarbeitung der Reaktionsgemische besonders einfach gestaltet.

Im übrigen nimmt man die Hydrierung wie üblich vor, also bei Temperaturen zwischen 20 und 150 °C und einem Druck von 20 bis 200 bar, wenn man weniger als 0,01 Gew.% Katalysatormetall (bezogen auf II) verwendet, und einem Druck von 1 bis 150 bar, wenn die Katalysatormenge größer ist als 0,01 Gew.%.

Mit Hilfe der erfindungsgemäßen Katalysatoren kann die Herstellung von ungesättigten Alkoholen durch selektive Hydrierung der entsprechenden α,β-ungesättigten Carbonylverbindungen in Gegenwart von Aminen wesentlich verbessert werden. Man erhält erheblich gesteigerte Raum-Zeit-Ausbeuten, verglichen mit der bekannten Arbeitsweise. Außerdem wird die Selektivität bezüglich der Verfahrensprodukte verbessert und über lange Betriebszeiten auf ihrem ursprünglich hohen Niveau gehalten, was vor allem für Duft- und Aromastoffe, wie dem Citronellal, von Bedeutung ist, da bei einer mit wirtschaftlich vertretbarem Aufwand vorgenommenen Reindarstellung des Produktes die Ausbeute an den gewünschten Verfahrensprodukten erheblich sinkt.

## Beispiel 1

### A. Herstellung des erfindungsgemäßen Katalysators

1 000 g pulverförmige Aktivkohle (BET-Oberfläche 750 $m^2/g$) wird mit einer Lösung von 140 g Rutheniumchloridhydrat in 2 000 g destilliertem Wasser getränkt und gut vermischt. Das erhaltene Material wird bei 80 bis 90 °C getrocknet und anschließend mit 15 g $Fe_2O_3$-Pulver trocken vermischt.

Das so erhaltene Katalysatorprodukt wird in einem gasdichten Drehrohrofen reduziert. Hierzu wird der Ofeninhalt mit einem Stickstoffstrom von 50 l/h eine Stunde lang gespült und anschließend in einem Wasserstoffstrom von 100 l/h innerhalb von 2 h auf 500 °C aufgeheizt. Nach 3 stündigem Reduzieren wird auf $N_2$ umgestellt und die Heizung abgeschaltet. Nach Abkühlung des Ofens wird das Produkt entnommen und in 1 000 g destilliertes Wasser eingetragen, so daß der fertige Katalysator einen Wassergehalt von 50 Gew.% hat.

Der trockene Katalysator enthält 5 Gew.% Ruthenium und 1 Gew.% Fe.

### B. Herstellung des erfindungsgemäßen Katalysators mit Reduktion des Katalysators bei Temperaturen zwischen 500 und 600 °C.

Es wird gearbeitet wie in A beschrieben, jedoch der Inhalt des Drehrohrofens nach dem Spülen mit dem Stickstoffstrom in einem Wasserstoffstrom von 100 l/h innerhalb von 2 h auf 600 °C aufgeheizt und anschließend 3 h reduziert.

### C. Vergleichsbeispiel

Herstellung eines Fe-dotierten Ru/C-Katalysators durch gleichzeitiges Tränken der Aktivkohle mit einer Ruthenium- und Eisensalzlösung und Reduktion des Katalysatorvorproduktes in ruhender Schicht.

1 000 g pulverförmige Aktivkohle (BET-Oberfläche 750 $m^2/g$) wird in einem Mischer mit einer Lösung von 140 g Rutheniumchloridhydrat und 40 g $FeCl_2 \cdot 4H_2O$ in 2 000 g destilliertem Wasser versetzt und 30 Minuten gut vermischt. Das erhaltene Material wird bei 80 bis 90 °C getrocknet.

Das so erhaltene Katalysatorvorprodukt wird in einen gasdichten, senkrecht stehenden Reduktionsofen eingefüllt, der über eine temperaturbeständige Siebplatte die Begasung des Katalysatormaterials gestattet. Der Ofeninhalt wird mit einem Stickstoffstrom von 50 l/h eine Stunde lang gespült und dann in einem Wasserstoffstrom von 100 l/h innerhalb von 2 h auf 500 °C aufgeheizt. Nach 3 stündigem

Reduzieren wird auf $N_2$ umgestellt und die Heizung abgeschaltet. Nach Abkühlung wird der Katalysator entnommen.

Der Katalysator hat einen Gehalt von 5 Gew.% Ru und 1 Gew.% Fe.

D. Vergleichsbeispiel

Herstellung eines Fe-dotierten Ru/C-Katalysators durch gleichzeitiges Tränken der Aktivkohle mit einer Ruthenium- und Eisensalzlösung und Reduktion des Katalysatorvorproduktes unter intensiver Durchmischung.

Das analog Vergleichsbeispiel 1C erhaltene Katalysatorvorprodukt wird in einem gasdichten Drehrohrofen reduziert. Hierzu wird der Ofeninhalt mit einem Stickstoffstrom von 50 l/h eine Stunde lang gespült und anschließend in einem Wasserstoffstrom von 100 l/h innerhalb von 2 h auf 500 °C aufgeheizt. Nach 3 stündigem Reduzieren wird auf $N_2$ umgestellt und die Heizung abgeschaltet. Nach Abkühlung des Ofens wird das Produkt entnommen und in 1 000 g destilliertes Wasser eingetragen, so daß der fertige Katalysator einen Wassergehalt von 50 Gew.% hat.

Der trockene Katalysator enthält 5 Gew.% Ruthenium und 1 Gew.% Eisen.

Beispiele 2 bis 6

Partielle Hydrierung von Citral

Jeweils 45 g reines Citral werden unter verschiedenen Bedingungen hydriert, wobei in allen Fällen Trägerkatalysatoren mit Aktivkohle als Träger verwendet werden, die in der Trockenmasse 5 Gew.% Ruthenium und die aus der Tabelle 1 ersichtliche Menge Eisen enthalten. Der Katalysator für die Beispiele 2A bis 2D ist jeweils der in den Beispielen 1A bis 1D hergestellte Katalysator. Der Katalysator für die Beispiele 3, 4, 5 und 6 wird analog Beispiel 1B hergestellt, der Katalysator für die Beispiele 3 (V) und 4 (V) ist ein käuflicher Ru/C-Katalysator. Die jeweiligen Reaktionsbedingungen sowie die Verfahrensergebnisse der einzelnen Versuche sind in der folgenden Tabelle 1 zusammengestellt. Vergleichsversuche werden hierbei mit (V) gekennzeichnet. Die Ausbeuten an Verfahrensprodukten wurden gaschromatographisch, die an Rückstand gravimetrisch bestimmt.

(Siehe Tabelle 1 Seite 7 f.)

Tabelle 1

| Vers. Nr. | Katalysator Ruthenium [Gew.%] 1) | Katalysator Eisen [Gew.%] 2) | Methanol [g] | Amin 3) [g] | Druck [bar] | Temp. [°C] | Zeit [h] | Umsatz 1) [%] | Ausbeute 4) Geraniol/ Nerol | Ausbeute 4) Citronellol | Rückstand |
|---|---|---|---|---|---|---|---|---|---|---|---|
| 2A | 0,04 | 0,56 | 32 | 16 | 50 | 100 | 6 | 100 | 96,5 | 2,3 | 1 |
| 2B | 0,04 | 0,61 | 32 | 16 | 50 | 100 | 5 | 100 | 96,2 | 3,1 | 1 |
| 2C(V) | 0,04 | 1,0 | 32 | 16 | 50 | 100 | 24 | 99,2 | 95 | 4,2 | 1 |
| 2D(V) | 0,04 | 0,84 | 32 | 16 | 50 | 100 | 21 | 98,7 | 94 | 5,3 | 1 |
| 3 | 0,02 | 0,56 | 32 | 16 | 100 | 50 | 4<br>5 | 100<br>100 | 96,2<br>95,8 | 2,8<br>3,2 | 1<br>1 |
| 3(V) 5) | 0,02 | - | 32 | 16 | 100 | 50 | 6<br>7 | 100<br>100 | 93<br>91,5 | 6,0<br>7,5 | 1 |
| 4 | 0,04 | 0,56 | 32 | 16 | 50 | 100 | 7<br>9 | 100<br>100 | 96,4<br>96,0 | 2,6<br>3,0 | 1 |
| 4(V) 6) | 0,1 | - | 32 | 16 | 50 | 100 | 8<br>9 | 100<br>100 | 93,4<br>91,8 | 6,1<br>7,2 | 1 |
| 5 | 0,04 | 0,56 | 32 | 16 | 20 | 100 | 15 | 99,2 | 96,3 | 3,0 | 1 |
| 6 | 0,04 7) | 0,56 | 32 | 16 | 50 | 100 | 8 | 99,8 | 96,1 | 2,8 | 1 |

1) bezogen auf eingesetzten Aldehyd
2) bezogen auf Gewicht des trockenen Katalysators
3) Trimethylamin
4) bezogen auf umgesetzten Aldehyd
5) entspricht Beispiel 3 aus DE-A 2 934 251
6) entspricht Beispiel 16 aus DE-A 2 934 251
7) Katalysator 7x rückgeführt

Beispiele 7 bis 9

Partielle Hydrierung von Citronellal

Jeweils 40 g reines Citronellal werden unter verschiedenen Bedingungen hydriert, wobei in allen Fällen Trägerkatalysatoren mit Aktivkohle als Träger verwendet werden, die in der Trockenmasse 5 Gew.% Ruthenium und die aus der Tabelle 2 ersichtliche Menge Eisen enthalten. Der Katalysator für die Beispiele 7, 8 und 9 wird analog Beispiel 1B hergestellt. Der Katalysator für Beispiel 7 (V) ist ein käuflicher Ru/C-Katalysator. Die Reaktionsbedingungen sowie die Verfahrensergebnisse sind in der folgenden Tabelle 2 zusammengestellt.

(Siehe Tabelle 2 Seite 9 f.)

Tabelle 2

| Vers. Nr. | Katalysator Ruthenium [Gew.%][1] | Eisen[2] [Gew.%][2] | Methanol [g] | Amin[3] [g] | Druck [bar] | Temp. [°C] | Dauer [h] | Umsatz[1] [%] | Ausbeute[4] Citronellol [%] | 3,7-Dimethyloctanol [%] | Isopulegol [%] | Rückstand |
|---|---|---|---|---|---|---|---|---|---|---|---|---|
| 7 | 0,02 | 0,56 | 32 | 16 | 50 | 100 | 6<br>8 | 99,8<br>99,9 | 98,2<br>97,9 | 0,3<br>0,6 | 0,3<br>0,3 | 1 |
| 7(V)[5] | 0,02 | – | 32 | 16 | 50 | 100 | 9<br>10 | 100<br>100 | 97,6<br>94,3 | 1,0<br>3,2 | 1,0<br>1,0 | 1 |
| 8 | 0,02 | 0,56 | 32 | 16 | 100 | 100 | 3,5<br>5 | 99,7<br>100 | 98,0<br>97,4 | 0,6<br>1,0 | 0,2<br>0,2 | 1 |
| 9 | 0,04 | 0,56 | 32 | 16 | 20 | 100 | 12 | 99,7 | 98,1 | 0,5 | 0,3 | 1 |

1) bezogen auf eingesetzten Aldehyd
2) bezogen auf Gewicht des trockenen Katalysators
3) Trimethylamin
4) bezogen auf umgesetzten Aldehyd
5) entspricht Beispiel 13 in Tabelle 2 der DE-A 2 934 251

## Beispiele 10 bis 11

Partielle Hydrierung verschiedener α,β-ungesättigter Aldehyde

Jeweils 40 g der in Tabelle 3 genannten Aldehyde werden unter den in dieser Tabelle angegebenen Bedingungen hydriert, wobei in allen Fällen Trägerkatalysatoren mit Aktivkohle als Träger verwendet werden, die in der Trockenmasse 5 Gew.% Ruthenium und die aus der Tabelle 3 ersichtliche Menge Eisen enthalten. Der Katalysator für die Beispiele 10 und 11 wird analog Beispiel 1B hergestellt, der Katalysator für die Beispiele 10 (V) und 11 (V) ist ein käuflicher Ru/C-Katalysator.

(Siehe Tabelle 3 Seite 11f.)

Tabelle 3

| Vers.Nr. | Aldehyd | Katalysator Ruthenium [Gew.%][1)] | Katalysator Eisen [Gew.%][2)] | Methanol [g] | Amin[3)] [g] | Druck [bar] | Temp. [°C] | Dauer [h] | Umsatz[1)] [%] | Ausbeute[4)] Alkohol | Ausbeute[4)] [%] | Rückstand [%] |
|---|---|---|---|---|---|---|---|---|---|---|---|---|
| 10 | 3,7-Dimethyl-nona-2,6-dien-1-al | 0,02 | 0,56 | 32 | 16 | 50 | 100 | 6 | 99 | 3,7-Dimethyl-nona-dien-1-ol | 96 | 1 |
| 10(V)[5)] | " | 0,02 | - | 32 | 16 | 50 | 100 | 9 | 99 | " | 94 | 1 |
| 11 | 3-Methyl-crotonal-dehyd | 0,02 | 0,56 | 32 | 16 | 50 | 100 | 7 | 99 | 3-Methyl-but-2-en-1-ol | 97 | 1 |
| 11(V)[6)] | " | 0,02 | - | 32 | 16 | 50 | 100 | 12 | 98 | " | 95 | 1 |

1) bezogen auf eingesetzten Aldehyd
2) bezogen auf Gewicht des trockenen Katalysators
3) Trimethylamin
4) bezogen auf umgesetzten Aldehyd
5) entspricht Beispiel 7 in Tabelle 3 von DE-A 2 934 251
6) entspricht Beispiel 4 in Tabelle 3 von DE-A 2 934 251

**Patentansprüche**

1. Ruthenium/Kohle-Katalysator für die Herstellung von olefinisch ungesättigten Alkoholen durch selektive Hydrierung der entsprechenden ungesättigten Carbonylverbindungen in der Flüssigphase, enthaltend neben 0,1 bis 10 Gew.% Ruthenium auf einem Kohlenstoffträger 0,1 bis 5 Gew.%, vorzugsweise 0,5 bis 1,5 Gew.% Eisen und hergestellt unter Beachtung der folgenden Maßnahmen

a) Dotierung des Katalysators mit Eisen erst nach erfolgter Imprägnierung mit der Rutheniumverbindung und

b) Reduktion des Katalysators mit Wasserstoff unter intensiver Durchmischung bei 400 bis 600 °C.

2. Ruthenium/Kohle-Katalysator gemäß Anspruch 1, bei dessen Herstellung die Reduktion des Katalysators mit Wasserstoff bei Temperaturen zwischen 500 und 600 °C vorgenommen wurde.

3. Verfahren zur Herstellung von Ruthenium/Kohle-Katalysatoren für die Herstellung von olefinisch ungesättigten Alkoholen durch selektive Hydrierung der entsprechenden Carbonylverbindungen durch Imprägnieren des Kohlenstoffträgers mit einer Rutheniumverbindung und anschließende Reduktion des Katalysators mit Wasserstoff, dadurch gekennzeichnet, daß man

a) den Katalysator nach Imprägnierung mit der Rutheniumverbindung mit 0,1 bis 5, vorzugsweise 0,5 bis 1,5 Gew.% an Eisen dotiert und

b) die Reduktion des Katalysators mit Wasserstoff unter intensiver Durchmischung bei Temperaturen von 400 bis 600 °C durchführt.

4. Verfahren zur Herstellung von Ruthenium/Kohle-Katalysatoren gemäß Anspruch 3, dadurch gekennzeichnet, daß man die Reduktion des Katalysators mit Wasserstoff bei Temperaturen zwischen 500 und 600 °C durchführt.

5. Verwendung der Ruthenium/Kohle-Katalysatoren gemäß Anspruch 1 zur Herstellung von ungesättigten Alkoholen der allgemeinen Formel Ia

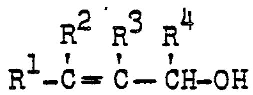

(Ia)

in der $R^1$ Wasserstoff oder einen organischen Rest und $R^2$, $R^3$ und $R^4$ Wasserstoff oder eine $C_1$-$C_4$-Alkylgruppe bedeuten, durch selektive Hydrierung der entsprechenden α,β-ungesättigten Carbonylverbindungen IIa

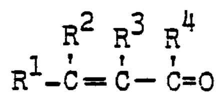

(IIa)

in der Flüssigphase in Gegenwart eines tertiären Amins.

6. Verbessertes Verfahren zur Herstellung von olefinisch ungesättigten Alkoholen der allgemeinen Formel Ia

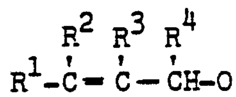

(Ia)

in der $R^1$ Wasserstoff oder einen organischen Rest und $R^2$, $R^3$ und $R^4$ Wasserstoff oder eine $C_1$-$C_4$-Alkylgruppe bedeuten, durch selektive Hydrierung der entsprechenden ungesättigten Carbonylverbindungen der Formel II

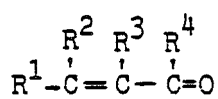

(IIa)

in der Flüssigphase mit Wasserstoff in Gegenwart von Edelmetallkatalysatoren und in Gegenwart von 5 bis 40 Gew.%, bezogen auf II, eines tertiären Amins, dadurch gekennzeichnet, daß man einen Ruthenium/Kohle-Katalysator gemäß Anspruch 1 als Edelmetallkatalysator verwendet.

**Claims**

1. A ruthenium-on-charcoal catalyst for the preparation of olefinically unsaturated alcohols by selective hydrogenation of the corresponding unsaturated carbonyl compounds in the liquid phase, which, in addition to from 0.1 to 10 % by weight of ruthenium on a carbon carrier, contains from 0.1 to 5 % by weight, preferably from 0.5 to 1.5 % by weight, of iron and is prepared by

a) modifying the catalyst with iron only after it has been impregnated with the ruthenium compound, and

b) reducing the catalyst with hydrogen at from 400 to 600 °C, with thorough mixing.

2. A ruthenium-on-charcoal catalyst as claimed in claim 1, in the preparation of which the reduction of the catalyst has been effected with hydrogen at from 500 to 600 °C.

3. A process for the preparation of a ruthenium-on-charcoal catalyst for the preparation of olefinically unsaturated alcohols by selective hydrogenation of the corresponding carbonyl compound, by impregnating the carbon carrier with a ruthenium compound and then reducing the catalyst with hydrogen, wherein

a) the catalyst is modified with from 0.1 to 5 % by weight, preferably from 0.5 to 1.5 % by weight, of iron after it has been impregnated with the ruthenium compound, and

b) the catalyst is reduced with hydrogen at from 400 to 600 °C, with thorough mixing.

4. A process for the preparation of a ruthenium-on-charcoal catalyst as claimed in claim 3, wherein the reduction of the catalyst is carried out with hydrogen at from 500 to 600 °C.

5. The use of a ruthenium-on-charcoal catalyst as claimed in claim 1 for the preparation of an unsaturated alcohol of the general formula Ia

$$R^1-\overset{R^2}{\overset{|}{C}}=\overset{R^3}{\overset{|}{C}}-\overset{R^4}{\overset{|}{C}}H-OH \qquad \text{(Ia)}$$

where $R^1$ is hydrogen or an organic radical and $R^2$, $R^3$ and $R^4$ are each hydrogen or $C_1$-$C_4$-alkyl, by selective hydrogenation of the corresponding α,β-unsaturated carbonyl compound IIa

$$R^1-\overset{R^2}{\overset{|}{C}}=\overset{R^3}{\overset{|}{C}}-\overset{R^4}{\overset{|}{C}}=O \qquad \text{(IIa)}$$

in the liquid phase in the presence of a tertiary amine.

6. An improved process for the preparation of an olefinically unsaturated alcohol of the general formula Ia

$$R^1-\overset{R^2}{\overset{|}{C}}=\overset{R^3}{\overset{|}{C}}-\overset{R^4}{\overset{|}{C}}H-O \qquad \text{(Ia)}$$

where $R^1$ is hydrogen or an organic radical and $R^2$, $R^3$ and $R^4$ are each hydrogen or $C_1$-$C_4$-alkyl, by selective hydrogenation of the corresponding unsaturated carbonyl compound of the formula II

$$R^1-\overset{R^2}{\overset{|}{C}}=\overset{R^3}{\overset{|}{C}}-\overset{R^4}{\overset{|}{C}}=O \qquad \text{(IIa)}$$

with hydrogen in the liquid phase in the presence of a noble metal catalyst and in the presence of from 5 to 40 % by weight, based on II, of a tertiary amine, wherein a ruthenium-on-charcoal catalyst as claimed in claim 1 is used as the noble metal catalyst.

**Revendications**

1. Catalyseur à base de ruthénium-carbone pour la préparation d'alcools oléfiniquement insaturés par hydrogénation sélective des composés carbonylés insaturés correspondants en phase liquide, contenant, outre 0,1 à 10 % en poids de ruthénium sur un support carboné, 0,1 à 5 % en poids, de préférence 0,5 à 1,5 % en poids de fer et préparé en veillant à prendre les mesures suivantes :

a) Dopage au fer du catalyseur seulement après que l'imprégnation avec le composé du ruthénium a été effectuée et

b) Réduction du catalyseur par l'hydrogène sous mélange énergique, à une température de 400 à 600 °C.

2. Catalyseur à base de ruthénium-carbone selon la revendication 1, dans la préparation duquel la réduction du catalyseur par l'hydrogène est effectuée à des températures comprises entre 500 et 600 °C.

3. Procédé pour la préparation de catalyseurs à base de ruthénium-carbone pour la préparation d'alcools oléfiniquement insaturés par hydrogénation sélective des composés carbonylés correspondants, par imprégnation du support carboné avec un composé du ruthénium, suivie de réduction du catalyseur par l'hydrogène, caractérisé en ce que :

a) l'on dope le catalyseur, après l'imprégnation par le composé du ruthénium, avec 0,1 à 5 %, de préférence 0,5 à 1,5 % en poids de fer et

b) l'on effectue la réduction du catalyseur par l'hydrogène sous mélange énergique à des températures comprises entre 400 et 600 °C.

4. Procédé pour la préparation de catalyseurs à base de ruthénium-carbone selon la revendication 3, caractérisé en ce qu'on effectue la réduction du catalyseur par l'hydrogène à des températures comprises entre 500 et 600 °C.

5. Utilisation des catalyseurs à base de ruthénium-carbone selon la revendication 1 pour la préparation d'alcools insaturés de formule générale Ia

$$R^1-\overset{R^2}{\overset{|}{C}}=\overset{R^3}{\overset{|}{C}}-\overset{R^4}{\overset{|}{C}}H-OH \qquad (Ia)$$

dans laquelle $R^1$ représente un atome d'hydrogène ou un radical organique et $R^2$, $R^3$ et $R^4$ représentent chacun un atome d'hydrogène ou un groupe alkyle en $C_1$ à $C_4$, par hydrogénation sélective des composés carbonylés α,β-insaturés correspondants IIa

$$R^1-\overset{R^2}{\overset{|}{C}}=\overset{R^3}{\overset{|}{C}}-\overset{R^4}{\overset{|}{C}}=O \qquad (IIa)$$

en phase liquide en présence d'une amine tertiaire.

6. Procédé amélioré pour la préparation d'alcools oléfiniquement insaturés de formule générale Ia

$$R^1-\overset{R^2}{\overset{|}{C}}=\overset{R^3}{\overset{|}{C}}-\overset{R^4}{\overset{|}{C}}H-O \qquad (Ia)$$

dans laquelle $R^1$ représente un atome d'hydrogène ou un radical organique et $R^2$, $R^3$ et $R^4$ représentent chacun un atome d'hydrogène ou un groupe alkyle en $C_1$ à $C_4$, par hydrogénation sélective des composés carbonylés insaturés correspondants de formule II

$$R^1-\overset{R^2}{\overset{|}{C}}=\overset{R^3}{\overset{|}{C}}-\overset{R^4}{\overset{|}{C}}=O \qquad (IIa)$$

en phase liquide par l'hydrogène en présence de catalyseurs à base de métal noble et en présence de 5 à 40 % en poids, par rapport à II, d'une amine tertiaire, caractérisé en ce qu'on utilise un catalyseur à base de ruthénium-carbone selon la revendication 1 en tant que catalyseur à base de métal noble.